# EUROPEAN PATENT SPECIFICATION

(11) **EP 3 833 975 B1**
(45) Date of publication and mention of the grant of the patent: **26.11.2025**
(21) Application number: 19852032.2
(22) Date of filing: 21.08.2019
(51) Int. Cl.: G01N 33/543, G01N 33/533, G01N 33/531

(54) **MULTIPLEX DEVICE UTILIZING LINEAR ARRAY WITH SLANTED TEST LINES ON A LATERAL FLOW STRIP OR MICROFLUIDIC DEVICE**
MULTIPLEXVORRICHTUNG MIT LINEARER ANORDNUNG MIT SCHRÄGEN TESTLINIEN AUF EINEM LATERAL-FLOW-TESTSTREIFEN ODER EINER MIKROFLUIDISCHEN VORRICHTUNG
DISPOSITIF MULTIPLEX UTILISANT UN RÉSEAU LINÉAIRE COMPRENANT DES LIGNES DE TEST INCLINÉES SUR UNE BANDELETTE À ÉCOULEMENT LATÉRALE OU UN DISPOSITIF MICROFLUIDIQUE

(30) Priority: 21.08.2018 US 201862720290 P
(43) Date of publication of application: 16.06.2021
(73) Proprietor: Intelligent Material Solutions, Inc., Princeton, NJ 08540 (US)
(72) Inventor: COLLINS, Joshua, E., Philadelphia, PA 19146 (US); BELL, Howard, Y., Princeton, NJ 08540 (US); CORSTJENTS, Paul, 2333 ZC Leiden (NL); GELUK, Annemieke, 2333 ZC Leiden (NL)
(74) Representative: FRKelly
(86) International application number: PCT/US2019/047432
(87) International publication number: WO 2020/041423

(56) References cited:
- US-A1- 2005 208 593
- US-A1- 2006 127 886
- US-A1- 2007 105 237
- US-A1- 2007 248 983
- US-A1- 2010 081 214
- US-A1- 2012 021 528
- US-A1- 2018 143 191

## Description

### TECHNICAL FIELD

The present disclosure is drawn to a method and system for multiplexed lateral flow assays, and specifically to a linear array of slanted test lines on lateral flow strip or equivalent microfluidic device, especially for use on readers which can only scan in a single direction (ID scanners).

### BACKGROUND

Lateral flow devices (LFDs) are a particular type of biosensor. Typically, the LFDs use test strips which comprise a porous membrane. The membrane creates and sustains the flow of any sample and reagents via, e.g., capillary action. Typically, the membrane holds specific recognition elements in defined zones of the membrane itself, laid out in a linear / serial fashion, which may be referred to as detection sites or zones. In cases where the recognition elements are specific antibodies the device is sometimes referred to as a 'lateral flow immunoassay ' (LFIA). The LFD works by generating a signal based on a reaction occurring within a detection zone involving the sample, the antibodies, and a suitable probe. Regarding the probe, suitable bioselective reagents are linked to micro- or nano-materials, including soluble stains and/or fluorophores, that provide the signal and enable the detection of the reaction occurring on the membrane. As many LFDs rely on a visual readout, labels are often colored micro- or nanoparticles.

LFDs have been around for decades. One of the first mass-marketed LFDs was the well-known pregnancy test, which determines pregnancy by measuring, e.g., the amount of a particular hormone in urine. Since that time, LDFs have found applications in numerous fields (e.g., food safety, environmental testing, veterinary, forensic, etc.) , due to benefits in simplicity, rapidity, cost-effectiveness, and the fact that neither technical expertise nor additional technological devices for reading the results are generally required, unless the quantified results are required.

However, LFDs offer some challenges. In particular, reproducibility can be challenging, and LFDs can be difficult to manufacture. Further, the more complicated the LFD, the more likely any reader technology used to quantify the results of the LFD will need multiple passes to provide an answer.

As such, improvements in LFDs are desirable.

US 2010/081214 A1 discloses an analyte detection apparatus having at least one reservoir area and a wicking membrane, wherein a labeled specific binding partner is impregnated on the reservoir area; and a region on the wicking membrane where at least one chemical component is immobilized.

US 2007/248983 A1 discloses a device for simultaneously, qualitatively or quantitatively identifying a number of analytes in a liquid sample. The device comprises a membrane with: a charging zone for applying the liquid sample; at least two indicator zones, which can interact with the analytes, and at least one absorption area, which absorbs the liquid after passing the indicator zones, whereby the indicator zones are located between the charging zone and an absorption area. The invention is characterized in that the flowing directions from the charging zone through the respective indicator zones to an absorption area (flow paths) are essentially parallel, and at least two different flow paths exist. The invention also relates to a method for identifying a number of analytes or the derivatives thereof in a liquid sample. The method consists of applying the sample to the charging zone of the membrane of the device, whereby this sample is present in an amount sufficient for causing the sample liquid to flow through the indicator zones toward the absorption area, and for causing the analytes or the derivatives thereof in the sample liquid to form a complex in the indicator zones.

US 2005/208593 A1 discloses a method and field portable apparatus for analyte detection using lateral flow immunochromatographic assays, where a single test sample may be simultaneously applied to multiple assays.

US 2012/021528 A1 discloses test device and method for determining the presence or absence of one or more analytes in a fluid sample, the test device including a support or member bearing a mark thereon, and a matrix or member containing a capture zone. In operation, an observation area in the test device becomes transparent, thereby allowing the user to view a mark that is present on a support that is disposed beneath the observation area. Typically, the mark on the underlying support is configured as a minus (-) sign. In the absence of analyte in the sample, the test device presents a negative result as a minus (-) signal. In the presence of analyte in the sample, however, the mark operates in concert with a perpendicular test line on the observation area to present a positive result as a plus (+) signal that is visible to the user.

### BRIEF SUMMARY

The invention sets out a method for multiplexed lateral flow assays according to claim 1. Advantageous embodiments are set out in the dependent claims.

### BRIEF DESCRIPTION OF THE DRAWINGS

Figure 1 is a schematic of a prior art LFD test strip.
Figure 2 is a schematic of an embodiment of a disclosed LFD test strip.
Figure 3A is a schematic illustrating a potential arrangement of test lines on a test strip.
Figure 3B is a schematic illustrating a potential arrangement of test lines on a test strip using inert protein lines between the test lines.
Figure 3C is a schematic illustrating a potential arrangement of test lines on a test strip that utilizes channels.
Figure 3D is a schematic illustrating a potential arrangement of test lines and control lines in a staggered formation.
Figure 3E is a schematic illustrating a potential arrangement of test lines on a test strip where there is separation between the top of one row of test lines and the bottom of another row of test lines.
Figure 3F is a schematic illustrating a potential arrangement of test lines and control lines in an aligned formation.
Figure 4A is a schematic illustrating an arrangement of the conjugate pad utilizing mixtures of dried conjugate prior to each test line.
Figure 4B is a schematic illustrating an arrangement of the conjugate pad utilizing a strip of a single dried conjugate prior to each test line.
Figure 5 is a schematic illustrating a test strip being read using multiple scans with a 2-dimensional scanner.
Figure 6A is a visual representation of an array of scans (showing scans 11-29) across a strip targeting IL-6, IP-10, IFN-y, IL-10, TNFα, and CCL4.
Figure 6B is a graph showing the signal (in relative fluorescent units) as measured when doing a 1-dimensional scan at one of the positions (step 16) shown in Figure 6A, indicating peaks for IL-6 (601), IP-10 (602), IFN-γ (603), IL-10 (604), TNFα (605), and CCL4 (606).

### DETAILED DESCRIPTION

The present disclosure can best be understood in comparison to modem LFD strips.

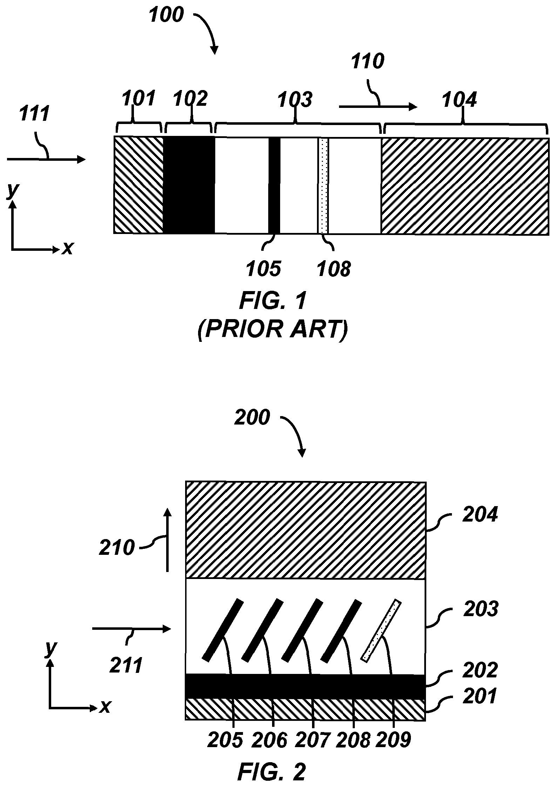

An example of a modem LFD strip can be seen in reference to Fig. 1. There, the strip (100) is a substrate that is typically longer in the direction of flow (x-direction) (110) than it is across (y-direction) (e.g., about 5 cm x 0.4 cm). The substrate can be divided into four general sections: a sample pad (101), a conjugate pad (102), a detection pad (103), and an absorbent pad (104).

The sample pad (101) receives a sample, either by putting a drop of the sample onto it, inserting the sample pad into the sample, or other known approach. Typically, there is a separate sample pad (101), and on top of it, closer to the, e.g., nitrocellulose detection pad (103), is the conjugate pad (102).

The sample pad (101) may be made of any material known to those of skill in the art.

The sample pad (101) may be a blood cell separation membrane.

The conjugate pad (102) stores detection molecules. Typically, the detection molecules provide signal or color change at the end of the test. These labeled-molecules have a high affinity to bind the target. Eventually, the label will provide the visual proof for presence of a target analyte. After a sample fluid is applied, it passes first through the conjugate pad, where the target analyte binds to labeled detection molecule to form a complex; this process develops the top half of what is commonly called an "Assay-Sandwich."

The conjugate pad (102) may be made of any material known to those of skill in the art.

In some instances, a portion of the sample pad (101) is used as a conjugate pad (102); in such cases, while there is no distinct sample pad (101) and conjugate pad (102) as illustrated in Fig. 1, the functions of each are still performed.

In some embodiments, no conjugate release pad (102) is used, for example, in research situations where an analyte already contains a conjugated target.

In some embodiments, the conjugate release pad (e.g., a glass fibre conjugate release pad) can be used as sample pad. This approach allows a user to premix sample and reporter before adding it to the strip as well as drying the reporter in the pad, and just adding sample; this situation delivers high flexibility for R&D purpose.

The detection pad (103) serves as the testing platform, where diagnostic schemes are developed. Prior art systems include two indicator lines: a test line (105) and a control line (108). The two indicator lines run across the test strip perpendicular to the direction of the sample flow (110). The complex formed on the conjugate pad is pulled by capillary flow toward a membrane incorporated into the detection pad, where the membrane has on it anchored (in the test lines) molecules designed to only capture the complex formed on the conjugate pad (e.g., capture molecules such as enzymes or antibodies). When this binding occurs, the "Assay-Sandwich" is complete and consists of an anchored capture molecule, an analyte, and a labeled detection molecule. The accumulation of such complexes on the test line is the basis for any Lateral Flow Assay.

The detection pad (103) may be made of any material known to those of skill in the art.

The absorbent pad (104) provides driving force through capillarity to pull a sample fluid along the test strip.

The absorbent pad (104) may be made of any material known to those of skill in the art.

Of particular note, in the prior art, the sample flow direction (110) is the same direction as the scan direction (111) used to read the test strip.

In use, there are typically two formats of test strips in current use: a competitive format and a sandwich format, both of which can be used as a multiplexed assay. Sandwich is the most used format (also in ELISA), this is where the target is capture by one antibody on the Test line and a second antibody coupled to a reporter will bind to another part of the captured antigen.

The competitive format is often used for low molecular weight compounds that cannot bind two antibodies simultaneously. Absence of color at test line is an indication for the presence of analyte while appearance of color both at test and control lines indicates a negative result. Competitive format LF As generally use one of three layouts. In the first layout, antigen in a sample solution and the one which is immobilized at test line on the strip compete to bind with labeled conjugate. In the second layout, labeled analyte conjugate is dispensed at the conjugate pad while a primary antibody to an analyte is dispensed at test line. After application of the analyte solution, a competition takes place between analyte and labeled analyte to bind with primary antibody at the test line. In the third layout, a new line (an antigen line) is used in between test and control lines for detection of C-reactive protein (CRP) in serum samples. This format involves a competition between the analyte in solution and analyte pre-dispensed on the new line. The new line is formed by dispensing CRP antibody solution followed by CRP solution. In case of very low concentration of CRP in a sample, most of the labeled conjugate molecules will remain unreacted and migrate to the antigen line and CRP present at antigen line will capture these labeled conjugates, resulting in an intense color at antigen line, while the rest of labeled conjugate will move to the control line and will produce relatively a light color. In case of very high concentrations, most of CRP molecules will be captured at the test line. In this case very few labeled conjugate molecules will be retained at antigen line. The lesser the color at antigen line, the higher the concentration of analyte.

The sandwich detection format is most often used for detection of more than one target species and assay is performed over the strip containing test lines equal to number of target species to be analyzed. It is highly desirable to analyze multiple analytes simultaneously under same set of conditions. Multiplex detection format is very useful in clinical diagnosis where multiple analytes which are inter-dependent in deciding about the stage of a disease are to be detected. Lateral flow strips for this purpose can be built in various ways, such as by increasing the length and number of test lines on a conventional strip, or by making other structures like stars or T-shapes.

An embodiment of the present disclosure can be seen in reference to Fig. 2. There, a test strip (200) can be seen that can also be divided into three sections: a sample pad (201), a conjugate pad (202), a detection pad (203), and an absorbent pad (204). These pads typically are on top of an underlying substrate that is used to provide some structure.

As discussed previously, in some embodiments, no conjugate pad (202) is used, for example, when analyte samples contain the conjugated molecules already. In other embodiments, the sample pad (201 and 202) functions as both the sample pad and the conjugate pad.

While the strip may have any dimensions, the strip is preferably shorter in the direction of flow (y-direction) (210) than it is across (x-direction) (e.g., 3 cm x 6 cm), with certain embodiments of the strip having dimensions where the ratio of length in the direction of flow-to-length across is > 1, and often between 1 and 5. Generally, dimensions are flexible but restricted to the size that a particular strip scanner (or strip reader/analyzer) allows. In some embodiments, the strip is between 30 and 50 mm in the y-direction. In some embodiments, the detection pad is between 20 and 30 mm in the y-direction, and the sample pad and absorption pad are each between 5 and 15 mm in the y-direction.

The sample pad (201) is configured to receive a sample, either by putting a drop of the sample onto it, inserting the sample pad into the sample, or other known approach.

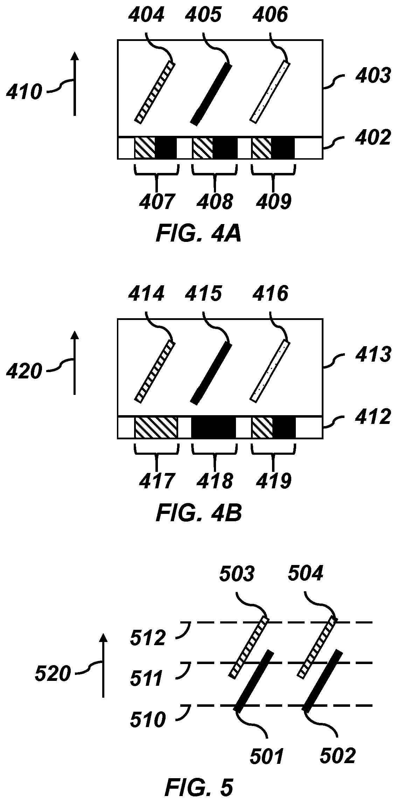

The portion of the sample pad (201) used as a conjugate pad (202) is configured to store labeled detection molecules that will bind with analytes of interest, as known to those of skill in the art. From a configuration standpoint, if a conjugate pad is utilized, it will generally be configured in one of two manners, as seen in Figs. 4A and 4B.

In Fig. 4A, the conjugate pad (402) is configured such that each slanted test line (404, 405) and flow control line (406) on the detection pad (403) has a corresponding section of the conjugate pad (407, 408, 409), such that a sample moving in the direction of flow (410) will pass over the corresponding section (e.g., 407) before moving over the test line (e.g., 404). In each corresponding section, there is a dried mixture of target-specific conjugates, typically presented as separate fragment for each of the target-specific conjugates used on the entire strip.

In Fig. 4B, the conjugate pad (412) is configured such that each slanted test line (414, 415) and flow control line (416) on the detection pad (413) has a corresponding section of the conjugate pad (417, 418, 419), such that a sample moving in the direction of flow (420) will pass over the corresponding section (e.g., 417) before moving over the test line (e.g., 414). Unlike what is seen in Fig. 4A, however, in this configuration, each corresponding section (e.g., 417) under a test line (414, 415) contains a single fragment of dried conjugate that will be used by the test line directly afterward (e.g., 414), in the direction of flow (420). The single flow control line here (416) uses a mixture of the target-specific conjugates, just as was done in Fig. 4A. If each test line has its own corresponding flow control line, then the flow control line could simply utilize the same corresponding section, or a corresponding section with the same single fragment of dried conjugate.

The absorbent pad (204) provides driving force through capillarity to pull a sample fluid along the test strip, as known to those of skill in the art.

The detection pad (203) of the disclosed lateral flow strip contains multiple slanted test lines (205, 206, 207, 208), and may contain one or more control lines (209). Preferably, between 2 and 10 test lines are utilized. As used herein to describe the disclosed embodiments, "test lines" can include shapes other than lines, including circles, ovals, rectangles, etc. The orientation and dimensions of lines is variable, depending on the dimensions of the strip which will be restricted by dimensions allowed with the scanner (strip reader/analyzer).

The underlying membrane may include nitrocellulose or equivalent known lateral flow or immuno-chromatography materials but may also include any microfluidic device alternative for immunochromatography, e.g., including micro-capillary devices.

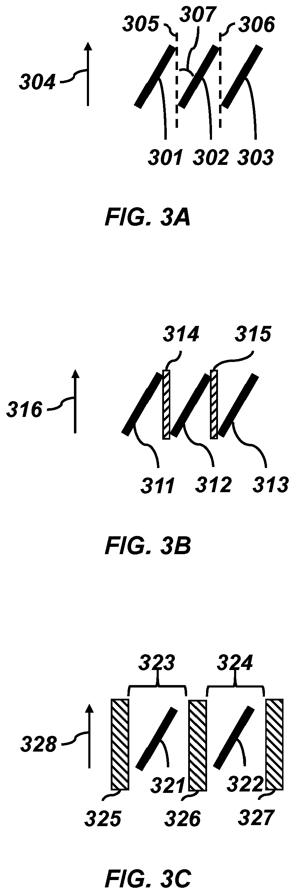

Referring briefly to Fig. 3A, in some embodiments of the strip, the angles and dimensions of the test lines (301, 302, 303) are selected such that no capture zone (test or control line) overlaps another capture zone in the direction of flow (304). Said differently, if imaginary lines (305, 306) were drawn on either side of a test line (e.g., 302) in the direction of flow (304), those imaginary lines would not cross the test lines to either side (e.g., 301, 303).

In some embodiments, regardless of the degree of overlap, the angle (307) between the test lines and the direction of flow (304) is less than or equal to 45 degrees. In some embodiments, the angle (307) is between 45 degrees and about 75 degrees.

Referring briefly to Fig. 3B, the slanted test lines (311, 312, 313) may or may not be divided by inert protein lines (or any other suitable material) (314, 315) to streamline flow in the direction of flow (316). A function of the inert protein (or any other suitable material) is to form artificial channels such that individual test lines more optimal interact with individual conjugates.

Referring briefly to Fig. 3C, the slanted test lines (321, 322) may be positioned within "channels" (323, 324) that are divided by hydrophobic or hydrophilic barriers (325, 326, 327), to prevent any portion of the sample from travelling between channels as it generally moves in the direction of flow (328).

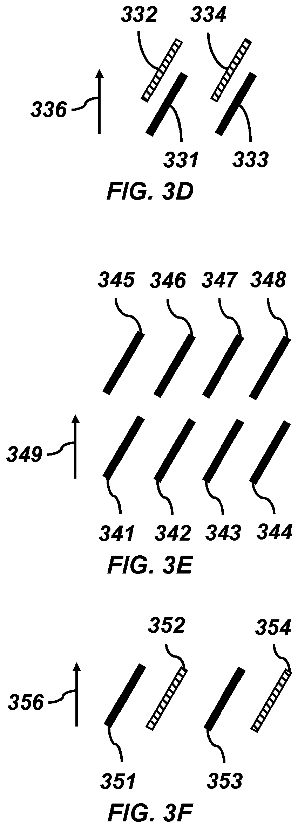

Referring briefly to Fig. 3D, each slanted test line (331, 333) and corresponding flow control line (332, 334) may be arranged in a staggered formation, where the flow direction (336) drives the sample fluid first through a slanted test line (e.g., 331), and then through an associated flow control line (e.g., 332). Said differently, a device that scans the strip from bottom-to-top will initially scan just the slanted test lines (331, 333), then will all of the various lines, and will eventually just scan the flow control lines (332, 334). However, this format was designed to allow a 1-dimensional scanner to scan through all test and flow control lines with a single scan. In other embodiments, there are more than two slanted lines placed above each other, then one would need a 2-dimensional reader to analyze the results. For example, if one has three test lines above each other in the same manner flow control line (332) is above test line (331), one can determine in a first scan the relation between Test lines 1 and 2, and in a second scan the relation between Test lines 2 and 3.

Referring briefly to Fig. 3E, in embodiments where there is a predetermined separation between the top of one row of test lines (341, 342, 343, 344) and the bottom of another row of test lines (345, 346, 347, 348), in the flow direction (349), all test lines can be evaluated individually.

Referring briefly to Fig. 3F, each slanted test line (351, 353) and corresponding flow control line (352, 354) may be arranged in an aligned formation, where the flow direction (356) drives the sample fluid first through a slanted test line (e.g., 351) and its associated flow control line (e.g., 352) in parallel. Said differently, all of the lines are aligned such that each scan perpendicular to the flow direction (356) will scan all of the lines.

Referring back to Fig. 2, the orientation of the lines will typically also be such that strips (200) can be read/analyzed in existing, portable and/or benchtop readers which only comprising scanning using x-axis movement (211) of the emission/excitation scan head, e.g., a linear array of slanted lines at substantially equal distance from the sample application pad. Some, but not all, of the embodiments of the disclosed test strip can be utilized with a **1-**dimensional scanner.

The test lines (204, 205, 206, 207) themselves may be comprised of any type of recognition molecule known in the art. This includes target-specific antibodies, nanobody (camelid (llama) or shark antibodies, including single domain antibodies), aptamer or any other target-specific capture/binding component, molecule, and/or biomolecule. These materials are known to those of skill in the art. The target may be a single molecule (e.g., caffeine, not necessarily be a single molecule of a defined composition and may include a specific class of molecules (e.g., polysaccharides including carbohydrates and hormones, etc.).

### Antibodies

Antibodies can be employed as biorecognition molecules on the test and control lines of lateral flow strip and they bind to target analyte through immunochemical interactions. Typically, an assay using antibodies is referred more specifically as a lateral flow immunochromatographic assay (LFIA). Antibodies are available against common contaminants, but they can also be synthesized against specific target analytes. Mice or other animals are immunized with target and secreted antibodies are subcloned and purified according to application. An antibody which specifically binds to a certain target analyte is known as primary antibody but the one which is used to bind a target containing antibody or another antibody is known as secondary antibody. The process of synthesizing an antibody against toxic analytes is challenging because of toxicity of injected analyte into animal body which may not be bearable by animal. Antibodies are generally produced from rat or mice and then applied to detect analytes from human samples. The affinity of any antibody toward a corresponding antigen is typically a concentration dependent factor, due to immune responses between them, and a reasonable response is typically observed in the range of 10⁷ to 10¹⁰ M⁻¹. Concentration of the target analyte is critical in deciding applicability of antibodies as biorecognition molecules. Limits of detections as low as nanomolar to picomolar range are

### Aptamers

As known in the art, aptamers are artificial nucleic acids generated by an in vitro process known as SELEX (systematic evolution of ligands by exponential enrichment). Aptamers have very high association constants and can bind selectively with a variety of target analytes Organic molecules having molecular weights in the range of 100-10,000 Da are outstanding targets for aptamers. Because of their unique affinity toward target molecules, very closely related interferences can be differentiated. They are preferred over antibodies due to many features which include easy production process, simple labeling process, amplification after selection, straightforward structure modifications, unmatched stability, reproducibility and versatility of applications.

DNA probes may also be employed for detection of DNA sequences related to different diseases, and genetic problems.

Labels are typically conjugated with a recognition molecule. Many materials can be used as a label, including nanoparticles (e.g., carbon nanoparticles, gold nanoparticles, selenium nanoparticles, silver nanoparticles, and rare earth nanoparticles), magnetic particles, quantum dots, soluble stains, organic or inorganic fluorophores, textile dyes, enzymes, liposomes and others. Typically, a label material should be selected that it is detectable at very low concentrations and retains its properties upon conjugation with the recognition molecule. In some embodiments, concentrations of labels above 10⁻⁹ M are optically detectable. Labels providing a direct signal are preferable, as opposed to those requiring additional steps to produce an analytical signal (e.g., enzymes that produce detectable product upon reaction with a suitable substrate).

In principle, any combination of humoral, cellular and pathogenic markers is possible. Various nanoparticle reporters can be utilized including rare earth nanoparticles, gold, silver, iron, etc. In a specific embodiment, rare earth nanoparticles of the composition, NaYF4:Yb,Tm with a 980nm to 800nm optical transition are used. In a different embodiment, down-converting nanoparticles such as NaYF4:Yb,Tm,Nd with excitation at 800nm and subsequent 900-1000nm emission may be used. Other reporters can be used that provide a qualitative signal such as gold nanoparticles, whereby a scanner is not utilized but instead the visual effect is detected by the human eye. The lateral flow nanocrystal provides quantitative analysis using signal ratios between test lines, test analysis (scanning) in single-pass portable scanners.

Preferably, rare earth particles are utilized. The crystals can be blended, mixed, coated, suspended into various ingredients. The surface chemistry of the crystals can be modified to be suspended into any polar and non-polar solutions.

Preferably, the rare earth particles are crystal phosphors that possess unique optical properties that can be detected at parts per billion levels, using one or more devices selected from a suite of field deployable/handheld and benchtop detection platforms capable of rapid identification of multiple optical signatures simultaneously. Optical signatures that can be analyzed include spectral wavelength and/or lifetime emission emitted from the crystal phosphors upon excitation with an appropriate wavelength. Crystal phosphor compositions possessing upconverting or downconverting optical transitions can be utilized in the assay formats as well.

Preferably, the crystal optical and magnetic properties of the rare earth particles are highly tunable. The rare earth particles are inert materials which can be modified with a variety of surface chemistries.

Potential crystal host compositions of rare earth particles can include but are not limited to halides such as NaYF₄, LiYF₄, BaYF₅, NaGdF₄, KYF₄, oxides such as Y₂O₃, Gd₂O₃, La₂O₃, oxysulfides such as Y₂O₂S, Gd₂O₂S, La₂O₂S. A selection of rare earth dopants can then be incorporated into the host lattice at varying concentrations. Single or multiple dopants can be incorporated into the host lattice giving rise to a unique optical property that can be readily measured using a paired optical detection device. Examples of dopant(s) and combinations are; YbEr, YbTm, YbHo, Er alone, Yb alone, Tm alone, NdTm, NdTmYb. The dopants can be incorporated into the host lattice anywhere from 0.02%-90% total rare earth doping concentration. For example, one composition could be NaYF₄:Yb(0. 7),Tm(0.02) with Yttrium (Y) comprising ~18% of the total rare earths, Ytterbium (Yb) 70%, and Thulium (Tm), 2%, which yields a 72% total rare earth doping concentration. The particle size range of these rare earth particles are optimally below 1 micron.

These rare earth particles can be further combined with various inorganic materials (e.g., gold and silver nanoparticles) and other organic or inorganic markers (e.g., rare earth chelates, Pd/Pt porphyrin dyes, etc.) that can be conjugated to the crystal surface providing, e.g., either enhanced plasmonic emissions or Fluorescence resonance energy transfer (FRET) / Luminescence resonance energy transfer (LRET) energy transfer conversions in order to, e.g., increase sensitivity and/or improve multiplexed detection capabilities.

Preferred rare earth particles have an extremely efficient and pure beta phase, crystalline structures with tunable morphologies, with particle sizes and optical properties that are substantially identical particle to particle. Such intersystem uniformity enables even single particle detection and very sensitive quantification capabilities due to the low signal to noise from the tunable spectral and lifetime properties as well as signal purity within the particle systems.

Suitable rare earth particles include the morphologically and size uniform, monodisperse phosphor particles described in US Patent 9,181,477 B2. Other rare earth particle compositions and architectures such as core-shell structures can be utilized as described in PCT Patent Application No. PCT/US2019/47397 filed August 21, 2019 and published as WO 2020/041402 A1 on February 27, 2020.

Generally, any label (or "reporter") technology may be utilized. Preferably, the label technology allows a quantitative readout using, e.g., a bench or portable scanner.

Once a test strip has been provided, a sample can be applied to the sample pad. The sample is then allowed to flow over the conjugate pad (102), which conjugates analytes in the sample with, e.g., a rare earth particle. The sample is allowed to continue flowing up the strip, and across the test lines on the detection pad (103), and on towards the absorbent pad (104). After passing over the test lines, the strip can be scanned in a direction perpendicular to the direction of flow. The scanner can illuminate the test line with at least one wavelength of light that the rare earth particle will absorb. The rare earth particle will then emit a different wavelength that can be detected by a sensor. The signal generated by the sensor when detecting that different wavelength relates to the concentration of the analyte in the sample.

In some embodiments, it may be useful to have different rare earth particles bind to each analyte. The scanner may be used to differentiate the various particles, using characteristics of the rare earth particle, including rise time and/or decay time at one or more wavelengths.

Some embodiments allow for the monitoring of the effect of treatment and status of the patient over time. In some embodiments, a patient could test themselves either using fingerprick blood, saliva or urine. In some embodiments, the analysis of the strip may preferably be sent, automatically, to a hospital, along with information correlating to the patient (e.g., patient name, ID number, etc.) and the patient could receive feedback in cases where some action is required.

To use, one embodiments starts with a sample is taken (e.g., a fingerprick to gather blood), and then the sample is diluted in an assay buffer. In some instances, the fingerprick blood can be utilized for direct measurement. The diluted sample is added to a disclosed LFA strip, and after some predetermined amount of time to allow for lateral flow (e.g., often 15-60 minutes), a scanner can be used to read the LFA strip. However, other approaches can be taken; sample preparation methods can vary depending on sample specimen. For example, urine may be utilized without addition of buffer.

Referring to Fig. 5, each strip may be scanned multiple times using 2D scanner motion, with each line of scans (510, 511, 512) occurring in a direction perpendicular to the direction of flow (520). Each scan line is typically given a sequential number, starting from the bottom of the lowest slanted line (501, 502) and proceeding towards the top of the highest slanted line (503, 504). In Fig. 5, a first scan (510) occurs near the bottom of the lowest slanted test lines (501, 502). The second scan (511) is shown occurring in the middle, crossing all of the slanted lines. The third scan (512) occurs near the top of the highest slanted flow control lines (503, 504). In Fig. 5, only 3 scans are shown. In some embodiments, between 3 and 50 lines are scanned for each LFA strip. In preferred embodiments, between 10 and 30 lines are scanned.

As an example, it is possible to make a determination of IgM vs. IgG when IgM is in excess of IgG. This approach requires a scanner with 2D scanner movement option. Excess IgM saturates (blocks) the downstream part of a PGL-I capture line. Using a 2D scanner, the scan line at which the IgG signals becomes visible relative to the scan line where the IgM line becomes visible determines the ratio between IgM and IgG.

In some embodiments, quantitative multiplex testing is accomplished using relative signal measurements - signal ratio between the different test lines. This means that the result does not have to be related to a (predetermined) standard curve.

As an example, a strip similar to that in Fig. 2 can be created to detect amounts of immunoglobulins G (IgG), and M (IgM), where the ratio can be used to discriminate secondary and primary infections of a particular type (e.g., Dengue Virus (DENV)). As the disclosed design provides a means for testing levels with no competition between test lines (since the sample does not flow over each test line in succession), when scanned, all that is needed to determine the ratio is the signal ratio between the two test lines - the signals do not need to be compared to a calibration curve.

This approach can be used in a wide range of applications. For example, human health applications may include, but are not limited to (i) a multiplex strip for detection and confirmation of infection (e.g. HIV: antibody and pathogenic marker) [humoral and pathogen]; (ii) multiplex strip for detection Schistosoma carbohydrates (e.g., to determine species type) [pathogen]; (iii) serological multiplex strip for detecting antibody subtype (e.g. infection status: IgG/IgM ratio) [humoral]; (iv) serological multiplex strip for various panels (e.g. diarrhea panel, respiratory panel) [humoral]; (v) combination of immuno drug, cellular disease marker, and anti-drug antibody (e.g. Crohn's) [medication, cellular and humoral]; (vi) combination of humoral marker and cellular markers (e.g., PGL-I and IP-10, leprosy) [humoral and cellular]; (vii) combination of cellular markers (e.g. TB signatures: Screen, Predict & Pediatric TB grants) [cellular]; (viii) combination of cellular markers for chronic infections such as Crohn's [cellular]; (ix) combination of disease markers in diagnosis of Traumatic Brain Injury; and (x) combination of disease markers for cardiac failure.

Agriculture applications may include, but are not limited to (i) combination of disease markers for infectious diseases of plants and animal livestock; (ii) combination of organismal (bacterial) markers for monitoring of both harmful and beneficial microbes for soil health; and (iii) combination of bacterial cellular markers, proteins or other antibody-specific targets for food-related human pathogens such as E. coli, salmonella, etc.

Water Quality Testing may involve, for example, a combination of markers for identification of pathogens in water such as Legionella, crypto giardia, and E. coli. CBRNE testing may include a combination of markers for identification of various CBRNE targets such as ricin, nerve agent, anthrax, radiation exposure, etc.

The multiplexing approach may include multiplex detection of various types of biomolecules: protein markers (including antibodies, host cellular response and pathogen derived biomolecules), carbohydrates (including glycoproteins), hormones, metabolites. In certain embodiments, the number of targets (level of multiplexing) starts at 2, but in principal no upper limitation. In preferred embodiments, the number of targets is between 2 and 20.

When considering clinical samples and standards, this method and system is applicable for serum, plasma, finger-stick blood (capillary blood), dried blood spots, stimulated peripheral blood T-cells (including Quantiferon supernatant), culture material, saliva (independent on collection method), sputum, nasal washes, urine, vaginal lavages, extractions of stool, biopsies, tissue materials. Further, it is applicable for samples, including biological samples, having received any type of sample preparation (including any possible purification, filtration, or concentration method); this includes dilution series of appropriate standards in any type of fluid. Thus, samples may include any/all body fluids suited for a particular specific diagnostic application, and material extracted or (partly) purified from any clinical specimen. Samples may or may not require dilution in assay buffer before application to the test device, and samples (after extraction or purification) may or may not require a pre-flow concentration step. Further, sample collection protocol may or may not require exact volume metering.

Antibody assays may involve utilizing a generic antibody optical reporter label (e.g., prot A, prot G, prot L) in a serological multiplex test for, e.g., antibody detection against infectious disease panel, detection of autoantibodies, or trough level determination of (panels of) immunotherapeutics and potential presence human antibodies against those.

One embodiment may use antibody isotype specific optical reporter labels (e.g. anti-IgM, anti-IgG) in a serological multiplex test to detect isotype specific antibodies against infectious diseases determine status of the disease.

In another embodiment, four test lines (for analytes CRP, IP-10, SAA, and Ferritin) and associated flow control lines were provided in a staggered arrangement in a multiplexed assay for the rapid detection of tuberculosis. The four analytes were detected with respective anti-antibodies conjugated to a UCP and dried into the conjugate release pad. At concentration of 1% and 10% serum in an assay buffer, samples for a patient with no tuberculosis exhibited no response or very faint response for IP-10, SAA, and Ferritin. CRP in the 1% sample was also faint. At concentrations of 1% serum for a patient with tuberculosis, the sample exhibited faint responses only for IP-10 and Ferritin. Thus, at 1% serum, a strong reading for CRP and SAA was an indicator of tuberculosis. At concentrations of 10% serum for a patient with tuberculosis, the sample did not exhibit any faint responses. Thus, using a 10% serum, a strong reading for IP-10, SAA, and Ferritin was an indicator for tuberculosis.

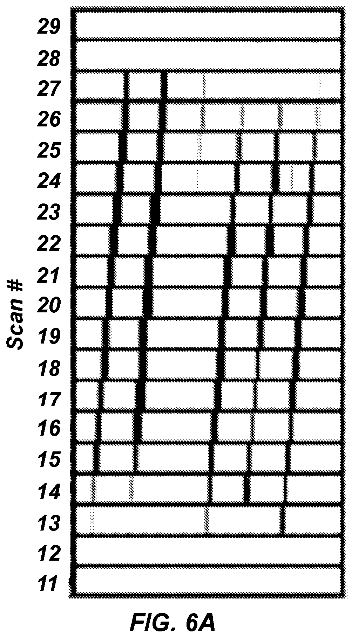

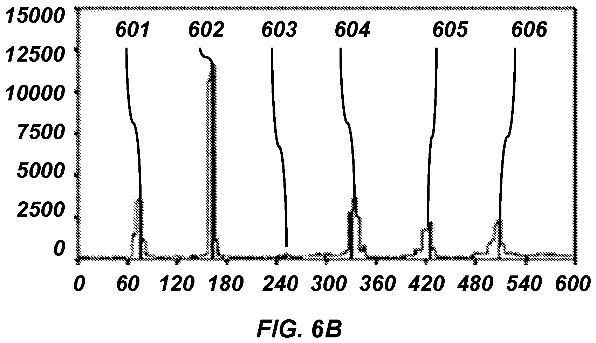

Referring to Figs. 6A and 6B, a 6-marker UCP-LFA slanted linear array for IL-6, IP-10, IFN-y, IL-10, TNFα, CCL4 was tested, with test lines arranged in that order from left to right across the strip. For the test, 50 µL of blood serum with 10 ng/mL of spiked antigen standard was used in final volume of 750 µL. After the sample flowed up the strip, a series of 30 scans were taken across the strip, from left to right. Scan 1 was across the bottom end of the test lines, and scan 30 was across the top end of the test lines. Fig. 6A shows the results of those scans. As seen in Fig. 6A, the scans generally detect signals starting at around scan line 13, growing in strength and then fading, with no signals being detected in scan line 28, and that the second test line, for IP-10, appears to be the strongest signal, while the third test line, for IFN-y, appears to be the weakest. This matches what is seek in the peak analysis of scan line 16, seen in Fig. 6B. Fig. 6B shows the signal (in relative fluorescent units) across the strip in the x-direction, as measured when doing a 1-dimensional scan across scan line 16. There, it is clear that at least some signal was received at each of the test lines: IL-6 (601), IP-10 (602), IFN-y (603), IL-10 (604), TNFα (605), and CCL4 (606). It is clear from Fig. 6B that IP-10 has the highest peak (here, indicating the highest concentration), IL-10 has the smallest peak (here, just above the level of detection), and the remaining four analytes have concentrations between those two.

## Claims

1. A method for multiplexed lateral flow assays, comprising the steps of:
providing a test strip for multiplexed lateral flow assays, comprisinga first section aligned with a bottom edge of the test strip, the first section configured to receive a sample containing an analyte, provide a plurality of conjugated rare earth particles capable of binding to the analyte, and allow the sample to flow in a direction towards a top edge of the test strip;
a second section in fluid communication with the first section, the second section comprising a plurality of test lines, the plurality of test lines forming at least one row of test lines, each test line arranged at an angle of 75° or less between the test line and the direction of flow, where at least one of the plurality of test lines is configured to capture the conjugated rare earth particle bound to the analyte;
a third section in fluid communication with the second section and aligned with the top edge of the test strip, the third section configured to absorb at least a portion of the sample after passing through the second section; and
wherein each row of the at least one row of test lines on the test strip is configured to be scanned in at a direction perpendicular to the direction of flow, and each such scan perpendicular to the flow direction will scan all lines in said row;
applying a sample to the first section;
allowing the sample to flow in a direction from the first section across the at least one test line in the second section; and
scanning the test strip in a direction perpendicular to the direction of flow,
the scanning including illuminating at least a portion of the test strip with at least one first wavelength of light and detecting a first signal emitted from a conjugated rare earth particle captured at a first test line and detecting a second signal from a conjugated rare earth particle captured at a second test line, the first signal being emitted at a wavelength different from the first wavelength; and
comparing the first signal to the second signal.

2. The method according to claim 1, wherein the first signal is transmitted to a hospital.

3. The method according to claim 1, wherein the conjugated rare earth particle comprises an up-converting nanoparticle.

4. The method according to claim 1, wherein the conjugated rare earth particle comprises a down-converting nanoparticle.

5. The method according to claim 1, wherein the plurality of test lines is configured in a single row.

6. The method according to claim 1, wherein the plurality of test lines is configured in plurality of rows, including a first row and a second row.

7. The method according to claim 6, wherein a top of the first row and a bottom of the second row are separated by a predetermined distance in the direction of flow.

8. The method according to claim 6, wherein the first row and second row are configured to be read by a 1-dimensional scanner.

9. The method according to claim 1, wherein the second section further comprises at least one flow control line.

10. The method according to claim 9, wherein the second section comprises an equal number of test lines and flow control lines.

11. The method according to claim 9, wherein the test lines and the flow control lines are in a single row.

## Patentansprüche

1. Verfahren für Multiplex-Lateral-Flow-Assays, das die folgenden Schritte umfasst:
Bereitstellen eines Teststreifens für Multiplex-Lateral-Flow-Assays, umfassend einen ersten Abschnitt, der an einem unteren Rand des Teststreifens ausgerichtet ist, wobei der erste Abschnitt dazu konfiguriert ist, eine Probe, die einen Analyten enthält, aufzunehmen, eine Vielzahl von konjugierten Seltenerdteilchen bereitzustellen, die in der Lage ist, sich an den Analyten zu binden, und zu ermöglichen, dass die Probe in einer Richtung zu einem oberen Rand des Teststreifens fließt;
einen zweiten Abschnitt, der in Fluidverbindung mit dem ersten Abschnitt steht, wobei der zweite Abschnitt eine Vielzahl von Testlinien umfasst, wobei die Vielzahl von Testlinien mindestens eine Reihe von Testlinien bildet, wobei jede Testlinie in einem Winkel von 75° oder weniger zwischen der Testlinie und der Fließrichtung angeordnet ist, wobei mindestens eine der Vielzahl von Testlinien dazu konfiguriert ist, das an den Analyten gebundene konjugierte Seltenerdteilchen einzufangen;
einen dritten Abschnitt, der in Fluidverbindung mit dem zweiten Abschnitt steht und an dem oberen Rand des Teststreifens ausgerichtet ist, wobei der dritte Abschnitt dazu konfiguriert ist, mindestens einen Teil der Probe zu absorbieren, nachdem sie den zweiten Abschnitt passiert hat; und
wobei jede Reihe der mindestens einen Reihe von Testlinien auf dem Teststreifen dazu konfiguriert ist, in einer Richtung senkrecht zu der Fließrichtung abgetastet zu werden, und wobei jede derartige Abtastung senkrecht zu der Fließrichtung alle Linien in der Reihe abtastet;
Auftragen einer Probe auf den ersten Abschnitt;
Ermöglichen, dass die Probe in einer Richtung von dem ersten Abschnitt über die mindestens eine Testlinie in dem zweiten Abschnitt fließt; und
Abtasten des Teststreifens in einer Richtung senkrecht zu der Fließrichtung,
wobei das Abtasten Beleuchten mindestens eines Abschnitts des Teststreifens mit mindestens einer ersten Lichtwellenlänge und Erfassen eines ersten Signals, das von einem an einer ersten Testlinie eingefangenen konjugierten Seltenerdteilchen emittiert wird, und Erfassen eines zweiten Signals von einem an einer zweiten Testlinie eingefangenen konjugierten Seltenerdteilchen beinhaltet, wobei das erste Signal mit einer Wellenlänge emittiert wird, die sich von der ersten Wellenlänge unterscheidet; und
Vergleichen des ersten Signals mit dem zweiten Signal.

2. Verfahren nach Anspruch 1, wobei das erste Signal an ein Krankenhaus übertragen wird.

3. Verfahren nach Anspruch 1, wobei das konjugierte Seltenerdteilchen ein aufwärts konvertierendes Nanopartikel umfasst.

4. Verfahren nach Anspruch 1, wobei das konjugierte Seltenerdteilchen ein abwärts konvertierendes Nanopartikel umfasst.

5. Verfahren nach Anspruch 1, wobei die Vielzahl von Testlinien in einer einzigen Reihe konfiguriert ist.

6. Verfahren nach Anspruch 1, wobei die Vielzahl von Testlinien in einer Vielzahl von Reihen konfiguriert ist, die eine erste Reihe und eine zweite Reihe beinhaltet.

7. Verfahren nach Anspruch 6, wobei eine Oberseite der ersten Reihe und eine Unterseite der zweiten Reihe in der Fließrichtung um einen vorbestimmten Abstand getrennt sind.

8. Verfahren nach Anspruch 6, wobei die erste Reihe und die zweite Reihe dazu konfiguriert sind, durch einen eindimensionalen Scanner gelesen zu werden.

9. Verfahren nach Anspruch 1, wobei der zweite Abschnitt ferner mindestens eine Fließkontrolllinie umfasst.

10. Verfahren nach Anspruch 9, wobei der zweite Abschnitt eine gleiche Anzahl von Testlinien und Fließkontrolllinien umfasst.

11. Verfahren nach Anspruch 9, wobei die Testlinien und die Fließkontrolllinien in einer einzigen Reihe liegen.

## Revendications

1. Procédé pour des analyses multiplexées à écoulement latéral, comprenant les étapes consistant à :
fournir une bandelette de test pour des analyses multiplexées à écoulement latéral, comprenant une première section alignée avec un bord inférieur de la bandelette de test, la première section étant configurée pour recevoir un échantillon contenant un analyte, fournir une pluralité de particules de terres rares conjuguées susceptibles de se lier à l'analyte, et permettre à l'échantillon de s'écouler dans une direction vers un bord supérieur de la bandelette de test ;
une deuxième section en communication fluidique avec la première section, la deuxième section comprenant une pluralité de lignes de test, la pluralité de lignes de test formant au moins une rangée de lignes de test, chaque ligne de test étant agencée selon un angle inférieur ou égal à 75° entre la ligne de test et la direction d'écoulement, dans lequel au moins l'une de la pluralité de lignes de test est configurée pour capturer la particule de terre rare conjuguée liée à l'analyte ;
une troisième section en communication fluidique avec la deuxième section et alignée avec le bord supérieur de la bandelette de test, la troisième section étant configurée pour absorber au moins une partie de l'échantillon après avoir traversé la deuxième section ; et
dans lequel chaque rangée de l'au moins une rangée de lignes de test sur la bandelette de test est configurée pour être balayée dans une direction perpendiculaire à la direction d'écoulement, et chaque balayage correspondant perpendiculaire à la direction d'écoulement balayera toutes les lignes de ladite rangée ;
appliquer un échantillon sur la première section ;
permettre à l'échantillon de s'écouler dans une direction allant de la première section à la deuxième section, en passant par l'au moins une ligne de test ; et
balayer la bandelette de test dans une direction perpendiculaire à la direction d'écoulement,
le balayage comprenant l'éclairage d'au moins une partie de la bandelette de test avec au moins une première longueur d'onde de lumière et la détection d'un premier signal émis par une particule de terre rare conjuguée capturée au niveau d'une première ligne de test et la détection d'un second signal en provenance d'une particule de terre rare conjuguée capturée au niveau d'une seconde ligne de test, le premier signal étant émis à une longueur d'onde différente de la première longueur d'onde ; et
comparer le premier signal au second signal.

2. Procédé selon la revendication 1, dans lequel le premier signal est transmis à un hôpital.

3. Procédé selon la revendication 1, dans lequel la particule de terre rare conjuguée comprend une nanoparticule à conversion ascendante.

4. Procédé selon la revendication 1, dans lequel la particule de terre rare conjuguée comprend une nanoparticule à conversion descendante.

5. Procédé selon la revendication 1, dans lequel la pluralité de lignes de test est configurée sur une seule rangée.

6. Procédé selon la revendication 1, dans lequel la pluralité de lignes de test est configurée en une pluralité de rangées, comprenant une première rangée et une seconde rangée.

7. Procédé selon la revendication 6, dans lequel une partie supérieure de la première rangée et une partie inférieure de la seconde rangée sont séparées par une distance prédéterminée dans la direction d'écoulement.

8. Procédé selon la revendication 6, dans lequel la première rangée et la seconde rangée sont configurées pour être lues par un scanner unidimensionnel.

9. Procédé selon la revendication 1, dans lequel la deuxième section comprend en outre au moins une ligne de régulation d'écoulement.

10. Procédé selon la revendication 9, dans lequel la deuxième section comprend un même nombre de lignes de test et de lignes de régulation d'écoulement.

11. Procédé selon la revendication 9, dans lequel les lignes de test et les lignes de régulation d'écoulement se trouvent sur une seule rangée.
